# EUROPEAN PATENT SPECIFICATION

(11) **EP 3 054 832 B1**
(45) Date of publication and mention of the grant of the patent: **01.08.2018**
(21) Application number: 14790676.2
(22) Date of filing: 09.10.2014
(51) Int. Cl.: A61B 1/04

(54) **ENDOSCOPY CAPSULE WITH SPAD ARRAY FOR DETECTING FLUORESCENCE EMITTED BY BIOLOGICAL TISSUE**
ENDOSKOPIEKAPSEL MIT SPAD-ARRAY FÜR DEN NACHWEIS DER VON EINEM BIOLOGISCHEN GEWEBE EMITTIERTEN FLUORESZENZ
CAPSULE D'ENDOSCOPIE COMPRENANT UN GROUPE DE SPAD POUR DÉTECTER UNE FLUORESCENCE ÉMISE PAR UN TISSU BIOLOGIQUE

(30) Priority: 10.10.2013 GB 201317968
(43) Date of publication of application: 17.08.2016
(73) Proprietor: The University Court Of The University Of Glasgow, Glasgow, Strathclyde G12 8QQ (GB)
(72) Inventor: CUMMING, David, Robert, Sime, Glasgow, Strathclyde G12 8QQ (GB); AL-RAWHANI, Mohammed, Glasgow, Strathclyde G12 8QQ (GB)
(74) Representative: Johnson, Richard Alan
(86) International application number: PCT/GB2014/053041
(87) International publication number: WO 2015/052523

(56) References cited:
- US-A1- 2002 014 595
- US-A1- 2006 202 129
- US-A1- 2009 137 876
- US-A1- 2013 015 331
- AL-RAWHANI MOHAMMED A ET AL: "Design and implementation of a wireless capsule suitable for autofluorescence intensity detection in biological tissues.", IEEE TRANSACTIONS ON BIO-MEDICAL ENGINEERING JAN 2013, vol. 60, no. 1, January 2013 (2013-01), pages 55-62, XP002733593, ISSN: 1558-2531

## Description

### FIELD OF THE INVENTION

The invention relates to fluorescence endoscopy, and in particular to an endoscopy capsule or pill for inspecting parts of the gastrointestinal tract that are inaccessible to conventional endoscopes.

### BACKGROUND TO THE INVENTION

Current medical technology permits visual inspection for signs of disease of the oesophagus and colon via endoscopy, and of the entire gastrointestinal (GI) tract by capsule-based sensors comprising a camera, illumination LED, radio transmitter, and battery pack. Capsule sensors are potentially capable of detecting ailments including cancer, abnormal blood vessel formation, and Crohn's disease within the small intestine [1]-[3]. Signs of disease, particularly early stage cancer, may however be missed in viewing the 7 to 9 hours' worth of images generated by the capsule [4].

Autofluorescence imaging (AFI) has been shown experimentally to be an effective means of detecting early signs of disease within the GI tract. It exploits the fact that cancerous intestinal tissue exhibits a considerably lower autofluorescent response than healthy tissue when excited by blue or UV light. This technique offers improved prospects for detecting GI diseases especially when combined with other imaging techniques such as white light imaging and narrowband imaging which assists in decreasing the false-positive rate of AFI [5], [6]. A commercial endoscope-based AFI system [6] is capable of accessing the oesophagus and colon; however, the high illumination intensity required by the charge-coupled device (CCD) sensor and consequent power consumption preclude implementation in a battery-powered capsule.

Autofluorescence endoscopes exploit variations in emitted fluorescence by tissues to detect early signs of cancer in the upper part and lower part of the GI tract [7]. A very high sensitive light detector coupled with a high power blue or ultra-violet excitation source are the two main components of such systems. For this technology to reach the small intestine part of the GI tract, a small swallowable pill is required. Unlike fluorescence endoscopes, pills must be powered from small button cell batteries, and are consequently constrained by the available charge and supply voltage.

The device may also be used for fluorescence imaging wherein an exogenous fluorophore has been deliberately introduced as a marker for cancer. One example is the circumstance where a fluorescently labelled antigen is introduced to a patient. The antigen preferentially binds to a tumour rendering more highly visible when visualised using a fluorescence sensitive camera [22].

For fluorescence technology to be fitted in such a constrained environment, a low power blue or ultra violet LED operating at low light intensities is used as the excitation source. To cope with the very low excitation light power delivered by the LED, an ultra-sensitive imager is required to measure the intensity of the extremely weak fluorescence emission. In fact this fluorescence is so weak that it is necessary to detect individual photons. Single Avalanche Photon Detectors (SPADs) have therefore been chosen as the light detector for the imager. However, For SPAD to operate in Geiger mode and count photons, it needs to be biased above its breakdown voltage. This voltage can vary from 10 V to few hundreds of volts according to the technology used to fabricate the detector. In commercial CMOS process such as 350nm, a fabricated SPAD can require up to 30 V bias voltage. In order to provide such high voltage from the 3 V power supply available from two SR44 batteries capable of fitting within a capsule, a charge pump is therefore required to convert the low supplied voltage to one that is high enough to bias a SPAD.

AL-RAWHANI MOHAMMED A ET AL: "Design and implementation of a wireless capsule suitable for autofluorescence intensity detection in biological tissues." discloses a a capsule forming part of the closest prior art.

### SUMMARY OF THE INVENTION

At its most general, one aspect of the present invention proposes an endoscopy capsule or diagnostic pill, which provides a means for reducing or minimising optical interference between an excitation optical signal and an emission (e.g. autofluorescence response) optical signal, which can improve the sensitivity of the image sensor.

According to a first aspect of the invention there is provided an endoscopy capsule for detecting fluorescence emitted by biological tissue, according to claim 1. Various aspects of the invention are defined in the dependent claims.

### BRIEF DESCRIPTION OF THE DRAWINGS

Embodiments of the invention are discussed below with reference to the accompanying drawings, in which:
Fig. 1 is an exploded perspective view of a wireless fluorescence endoscopy capsule that is an embodiment of the invention;
Fig. 2 is a perspective view of the wireless fluorescence endoscopy capsule of Fig. 1 when assembled;
Fig. 3 is a system block diagram illustrating the electronic configuration of the wireless fluorescence endoscopy capsule of Fig. 1 and its radio link to an external base station;
Fig. 4 is a block diagram of a SPAD array imager chip used in the wireless fluorescence endoscopy capsule of Fig. 1;
Fig. 5 is a block diagram illustrating how each SPAD pixel in the array is time-gated in order to manage the driving power of the charge pump;
Fig. 6 is a schematic view of a charge pump suitable for biasing a 32 × 32 SPAD array;
Fig. 7 is a schematic view of a high voltage non-overlapping clock generator used in the charge pump of Fig. 6;
Fig. 8 is a schematic diagram of a single SPAD pixel in the array and its readout circuitry.
Fig. 9 is a graph showing measured photon detection probability of a SPAD pixel in the array;
Fig. 10 is a graph showing a measured voltage and current response of the charge pump when connected to the SPAD array as function of count rate;
Fig. 11 is a pair of graphs showing experimental results of the integration of the charge pump and the array SPAD;
Fig. 12 is a timing diagram for operating the wireless fluorescence endoscopy capsule of Fig. 1;
Fig. 13 is a graph showing the spectral characteristic of the fluorescence interference cube mounted in the wireless fluorescence endoscopy capsule of Fig. 1;
Fig. 14 is a pair of measurement results from the SPAD array sensor, respectively corresponding to a fixed noise signal and a drop of fluorescein isothiocyanate (FITC) fluorophore in an excited state;
Fig. 15 is a set of measurement results obtained from the SPAD array sensor using cancer phantoms;
Fig. 16 is a graph showing the variation in count rate with flavin adenine dinucleotide (FAD) fluorophore concentration in the presence and absence of haemoglobin;
Fig. 17 is a graph that compares measured autofluorescence emission obtained from illuminating a sample of porcine small intestine with a reflection response; and
Fig. 18 is a exploded cross-sectional view of a prism/filter arrangement used inside the capsule of Fig. 1.

### DETAILED DESCRIPTION; FURTHER OPTIONS AND PREFERENCES

Autofluorescence from healthy tissues in the GI tract exhibits a spectrum between 470 and 770 nm when excited with a 425 ± 52 nm light source [5, 6, 8]. The resulting autofluorescence is attributed to a number of endogenous fluorophores within the living tissue. The main contributor to the autofluorescence emission is flavin adenine dinucleotide (FAD) [9], which displays the most substantial and measurable autofluorescent intensity. FAD's peak excitation wavelength is 450 nm; hence, a 460-nm LED excitation source (460 nm is the closest commercially available miniature LED wavelength) in combination with a narrow band pass filter is used, so as to minimize unwanted noise due to autofluorescence by other fluorophores. FAD exhibits an autofluorescence peak at 520 nm [6, 10]; hence, a long-pass filter with a cut off wavelength of 515 nm is placed over the detector to further reduce noise from the autofluorescence of other molecules.

Autofluorescence emission from living tissue is weak and requires very sensitive light detectors and/or powerful illumination. Photomultiplier tubes offer high sensitivity, but bulk and the very high voltage required prevent use in a capsule. CCDs are compact and require lower power and voltage; however, the small batteries suitable for use in a capsule sensor are incapable of powering the intense illumination required [5, 8, 11].

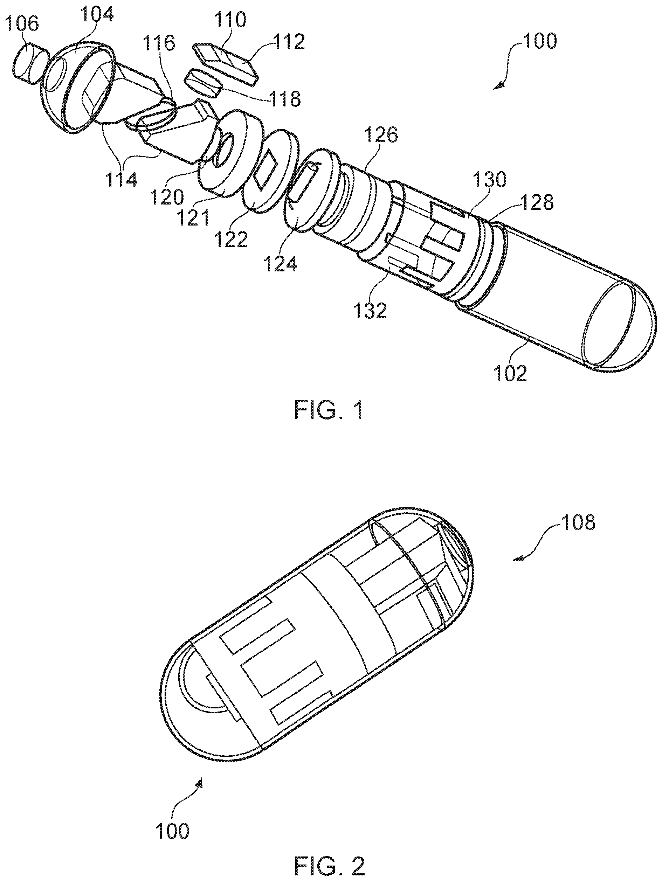

Fig. 1 shows an exploded perspective view of an endoscopy capsule 100 that is an embodiment of the invention. The components of the capsule are housed in a transparent casing 102, which is closed by a domed cap 104. The casing may be formed from plastic or glass, e.g. borosilicate glass which has a visible light transmission of greater than 92%. In this case, the capsule is a cylinder with rounded ends as is conventional. Other shapes of capsule may be used.

The domed cap 104 has an achromatic lens 106 mounted in it. As shown in Fig. 2, the lens 106 is arranged to both direct an excitation (illumination) signal towards and collect an input (response) optical signal from an illumination/detection region 108.

The excitation signal is generated by a blue light emitting diode (LED) 110 mounted on a printed circuit board 112 mounted along the side of the capsule. The output light from the LED 110 is directed into an interference filter cube or prism 114. The function of the interference filter cube 114 is to prevent unwanted leakage of the excitation signal, especially to prevent the excitation signal from leaking on to the image sensor, where it may cause errors in the detected signal.

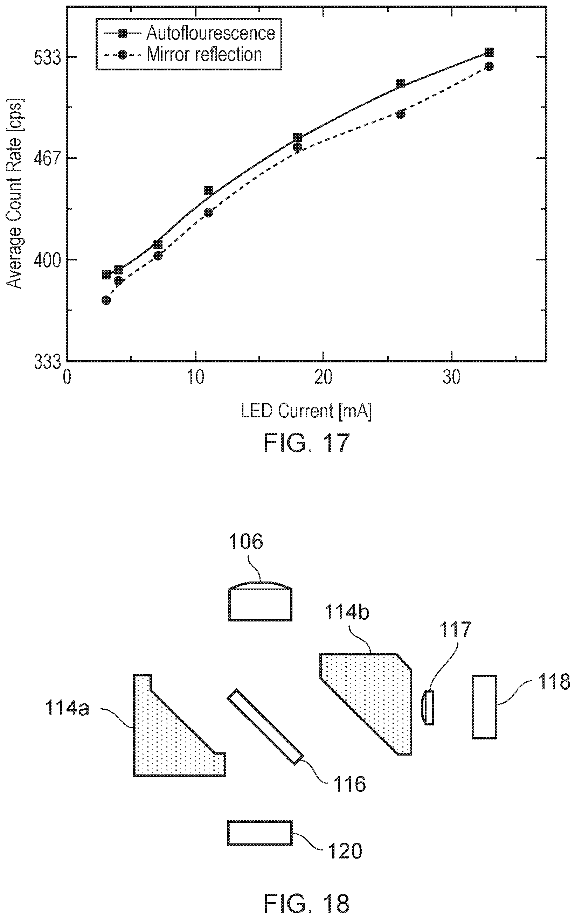

Fig. 18 shows the component parts of the prism used in the capsule imager. Two solid housing parts 114a, 114b define the structure of the prism. The device has front lens 106 and an angled beam splitter 116. The housing parts 114a, 114b encase the beam splitter 116. The beam splitter 116 ensures that the input optical signal reaches the image sensor.

In order to improve the ability of the device to differentiate between the excitation signal and an input signal resulting from an autofluorescent effect, a first band pass filter 118 is mounted after the illumination Light Emitting Diode (LED) 110 to restrict the output wavelengths to be in the ultraviolet range less than 480 nm. In this embodiment, the first band pass filter 118 sets a pass band of 420-480 nm for the excitation signal. A light source lens 117 is used to reduce the illumination angle of the LED and therefore minimise the possibility of crosstalk arising from direct illumination of the SPAD detector by the LED.

A second band pass filter 120 is mounted before the SPAD detector to allow only green light of wavelength 500-570 nm to illuminate the detector.

The interference filter cube 114 is physically separated from the image sensor by an annular spacer 121. The image sensor 122 itself comprises an application-specific integrated circuit (ASIC) on a PCB. The ASIC implements an 32 × 32 array of single-photon avalanche diodes (SPADs), a charge pump that enables the SPAD array to operate from a 3 V battery, and a multiplexer that is arranged to manage the power delivery to the SPADs. The ASIC includes data counters which log detection events in the SPAD array. These components and there functions are discussed in more detail below.

The image sensor 122 is in communication with a field-programmable gate array (FPGA) controller 124 which is arranged to receive the data output from the image sensor 122 and process it into a form suitable for transmission output for the capsule.

In this embodiment, the power source for the capsule is a 3 V battery 126, e.g. formed from a pair of 1.5 button cells (e.g. SR44 alkaline cells).

To transmit data out of the capsule to a remote location, the capsule includes transmitter circuitry 128 and a flexible thin film antenna 130 that is mounted on a support tube 132. The transmitter circuitry 128 may be an radiofrequency (RF) transmitter, e.g. which emits in the ultra high frequency (UHF) band. The support tube 132 may have a diameter large enough to receive the battery 126, FPGA controller 124 and image sensor 122. This enables a more compact assembly, as shown in Fig. 2.

The assembled capsule is shown in Fig. 2. The capsule may have a length dimension of less than 50 mm, e.g. 45 mm or less. The maximum diameter across the body of the capsule may be less than 20 mm, e.g. 16 mm or less.

Overall, the system operates as follows. Intestinal tissue emits photons in response to LED illumination, the SPAD pixels in the array generates a train of pulses in proportion to light intensity, the number of pulses within a fixed period is then counted, and the counter value and address of the pixel is then transmitted via ultrahigh frequency (UHF) radio to an external receiver/data logger.

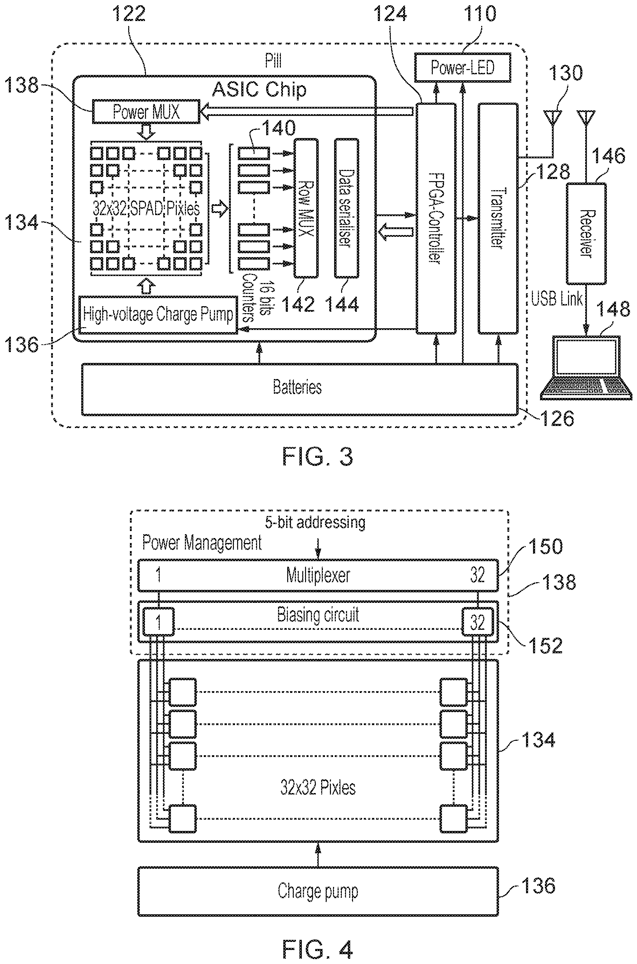

Fig. 3 is system block diagram illustrating the electronic configuration of the capsule. The components in common with Fig. 1 are given the same reference numbers and are not discussed again. Fig. 3 shows that the battery 126 supplies power to the LED 110, the transmitter 128, the FPGS controller 124 and the image sensor 122.

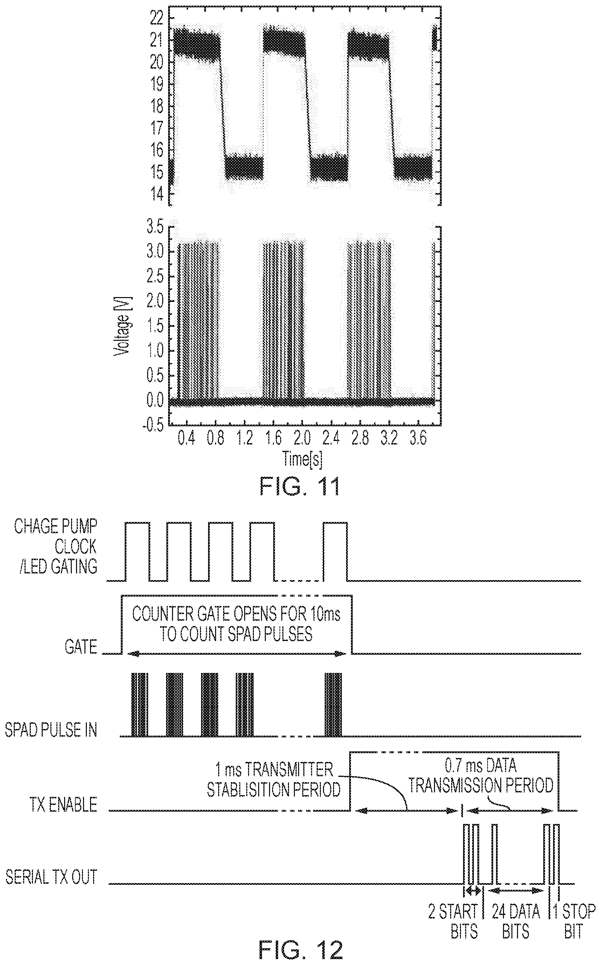

The image sensor 122 includes a 32 × 32 SPAC array 134, which receives power from a high-voltage charge pump 136 in a multiplexed manner under the control of a power management module 138. The FPGA controller 124 may include a timing circuit to control overall operation of the capsule (see Fig. 12) .

The output of the SPAD array 134 is communication to a set of 16 bit counters 140, which log the SPAD detection events. The set of counters 140 are polled by an output multiplexer 142 to generate a detection signal that is communicated to the FPGA controller via a data serialiser 144. The detection signal is then processed into a form suitable for wireless transmission to a remote receiver 146, which may be associated with a computer 148 for analysing the signal.

The image sensor chip design and charge pump design are now discussed in more detail.

A SPAD is a PN-junction avalanche photodiode that is reverse-biased above its breakdown voltage to operate in Geiger mode. At such high bias voltage, an electric field within the active region of the junction is created. Once hit by a single photon, an avalanche breakdown is initiated, creating a large enough current pulse to be readily detected by sensing circuitry. The self-sustaining avalanche process is quenched by reducing the bias voltage, so each photon generates a short pulse. Quenching process can be achieved either by connecting a resistor load externally or by integrating an active load such as an NMOS channel transistor.

At every avalanche event, the SPAD discharges its internal capacitance and other parasitic capacitances associated with its anode. In sub-micro CMOS processes, the sum of these capacitances can be less than a picofarad in cases where the quenching element is integrated [12, 13]. This limits the amount of discharged current following each avalanche process. Therefore reduces the delivered power supply required for biasing. An average SPAD size can draw a few micro-amps from its bias supply [12, 14]. However, for an array of SPADs, the amount of current required is multiplied by the number of SPADs. This increases the current demand to few milliamps for a 1,000 pixel array. In order to minimise the current required, the chip proposed herein has employed a power management technique to efficiently distribute the limited current that can be delivered by the battery via the charge pump.

The chip proposed herein is designed and fabricated using a high voltage, H35 CMOS process provided by Austria Microsystems. Fig. 4 shows a block diagram of the proposed chip which incorporates a 32 × 32 SPAD array 134, a high voltage charge pump 136 for providing the required high-bias voltage and a power management circuit 138 to control the distribution of the high-biasing voltage from the charge pump. The chip uses a "rolling shutter" readout arrangement, in which one 32-pixel column at a time is powered by the charge pump 136 via a column multiplexer and the pulse output from that column's SPADs is streamed at 32 row outputs. The power management block consists of a 5-to-32 multiplexer 150 and 32 conditioning circuits 152 for providing bias voltage to the SPAD's readout circuitry.

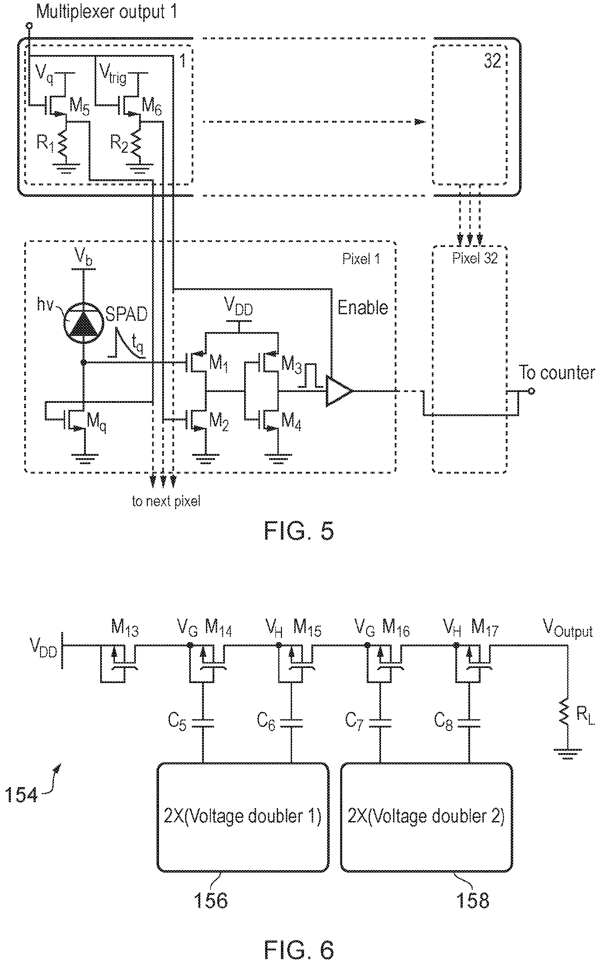

The mechanism of multiplexing the high-bias voltage provided by the charge pump can be understood by explaining the operation principle of a single SPAD pixel. As shown in Fig. 5, the SPAD anode is connect to n-channel transistor *M_{q}* which is the quenching element for every SPAD. When *M_{q}* is on (i.e. above threshold voltage) it acts as a resistive load.

At high bias voltage *V_{b}* set above the breakdown voltage, an avalanche breakdown occurs due to a photon impact. This forces the SPAD's capacitance to discharge rapidly, causing a significant current flow through the SPAD. Consequently, at the SPAD's anode, a voltage potential rises rapidly forcing the voltage across the SPAD's terminal to drop below the break down voltage and hence quenching the avalanche process. This process will continue for every photon striking the active area of the SPAD generating a train of triangular shape pulses with a decaying time *t_{q}.* As illustrated in Fig. 5, these pulses are passed through an inverting comparator formed by CMOS transistors *M*₁, *M*₂, where *M*₂'s *V_{trig}* input determines the trigger threshold. The inverter *M*₃, *M*₄ provides current gain; hence, the response is read nondestructively and converted to a digital pulse capable of driving the input of a tri-state multiplexer.

Multiplexing the *V_{b}* between the array columns is achieved by time-gating *M_{q}*. All SPADs in a single column can be set to idle mode by setting their *M_{q}* to zero and activated when set back to *V_{q}*. Furthermore, time gating *V_{trig}* and setting the tri-state logic to power-down mode contributes to the power management scheme and helps reducing total power consumption of the image sensor.

The time-gated *V_{q}* is obtained through a first controlled voltage divider *R*₁, *M*₅. The time-gated *V_{trig}* is obtained via a second controlled voltage divider *R*₂, *M₆*. Every output of the multiplexer is connected to one column of the array that is addressed by 5 bit address code supplied externally by the FPGA controller 124.

SPADs fabricated in the H35 CMOS technology have shown a break down voltage 15-30 V range. For such SPADs to operate in Geiger mode, an excess voltage *Vₑₓ* of 2 to 5 V has to added on top of the breakdown voltage. Therefore, a charge pump that is capable to provide a sustained required voltage from a 3 V power supply is desired. Furthermore, the charge pump has to be capable of delivering substantially few hundreds of micro-amps, required to bias one column of 32 SPADs. A Dickson charge pump cascades a number of pumping stages is usually sufficient to generate an output voltage that is few times higher than the input voltage. However, when number of stages is high, a standalone Dickson configuration is not an efficient choice, especially for low power applications. Due to the voltage drop across the diodes or diode-connected transistors in Dickson charge pumps, high-voltage non-overlapping clocks are used in the invention to improve the overall efficiency. The charge pump proposed herein is a combination of a multi-stage Dickson charge pump with multi-stage voltage doublers.

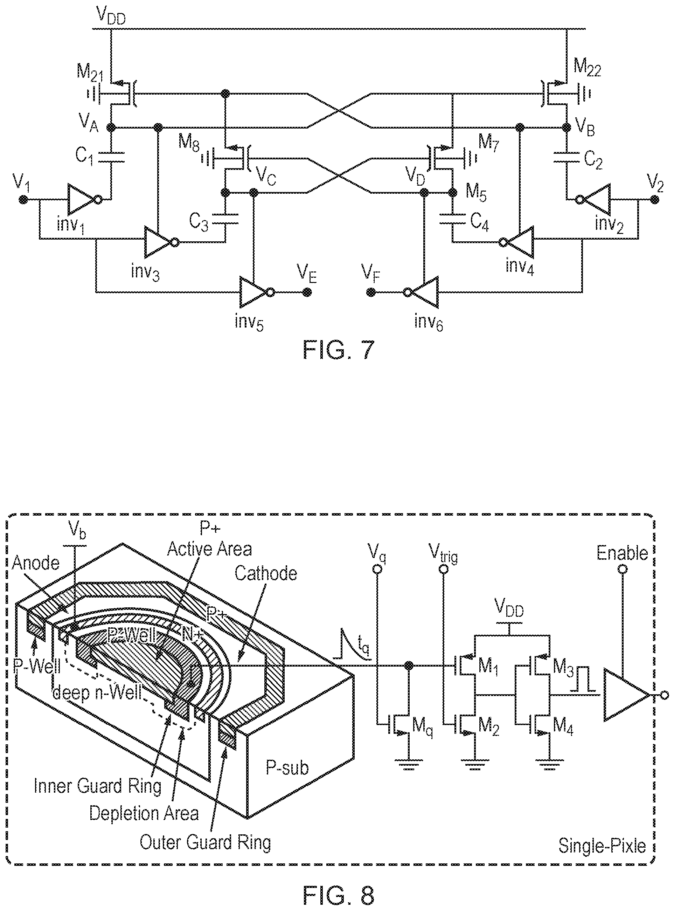

Fig. 6 shows a block diagram of the charge pump system used in the present embodiment. It consists of a five stage Dickson charge pump. As shown in Fig. 7, the circuit is clocked by non-overlapping clock signals *V*₁, *V*₂ which swing between 0 and *V_{DD}* (3 V) provided by two 2× voltage doublers 156, 158. At nodes *V_{A}* and *V_{B}* the corresponding voltage swing is between *V_{DD}* and *2V_{DD}. V_{A}* and *V_{B}* power high voltage inverters inv₃ and inv₄ via voltage doubles *M*₂₁, *C*₁ and *M*₂₂, *C*₂ respectively so that voltages *V_{C}* and *V_{D}* swing from *2V_{DD}* to *4V_{DD}. V_{C}* and *V_{D}* similarly power inverters inv₅ and inv₆ to generate non-overlapping clocks *V_{E}* and *V_{F}* which swing between ground and *4V_{DD}.* The Dickson charge pump 154 [15] comprising transistors *M*_{13,} *M*₁₄, *M*₁₅, *M*₁₆ and *M*₁₇ then accumulates the total voltage from the two 2× voltage doublers 156, 158 to produce an output *V*_{Output} which is ideally 17*V_{DD}* (51 V). Cadence Spectre simulations indicated that a single cell will generate only 40.4 V due to internal losses [16, 17].

All transistors used for designing the charge pump are high voltage devices that can tolerate up to 22 V between the drain and the source of the device. The charge pump was designed to use external capacitors *C*₅, *C*₆, *C*₇ and *C*₈. The non-overlapping clocks were designed to be derived from a single clock supply that can be generated off chip. Adjusting the load and the frequency of the charge pump care used vary the output voltage and consequently optimise SPAD sensitivity, thus avoiding the additional complexity of a separate voltage regulator.

Fig. 8 shows a schematic cut away perspective view of a single SPAD pixel of the SPAD array of the embodiment discussed above, together with the connections to the associated circuitry. The cross-section view through the centre of the SPAD shows a guard ring of low-doped material used to reduce the electric field at the edges and corners. The active area is 10 µm diameter, and the overall SPAD area including guarding is 30 µm diameter.

The curvature around the edges of a standard CMOS planar p-n junction tends to cause a higher electric field than in the main active area; hence, the edges will break down at lower voltages than the main area [18]. A guard ring of a lower doped material (as used in the anode) [19-21] is diffused around the edges of the active area, reducing the electric field at the edges and thus preventing premature breakdown. Higher electric field also occurs in the corners of a rectangular p-n junction, a problem avoided in the embodiment by using a circular SPAD layout.

The SPAD array and charge pump discussed above can be laid out on a 3.7 mm × 2.5 mm chip die. The charge pump occupies 0.8 mm × 1.75 mm and the SPAD array and its associated circuitry occupies 2.2 mm × 2.2 mm. The charge pump was surrounded by a deep well guard ring in order eliminate any effect that can be caused by leakage currents injected into the substrate due to charge pump's continuous switching nature [15]. The cathodes of all the 1024 SPADs were connected to a single input pad in the chip. The output of the charge pump is connected to an output pad. This allowed testing and evaluation of the array and the charge pump separately.

Experimental testing of the SPADs array used in this study showed that each SPAD exhibited an average breakdown voltage of 19 V. In order to bias the array SPAD to operate in Geiger mode, the SPAD was biased as 22.5 V with an excess voltage *Vₑₓ* of 3.5 V.

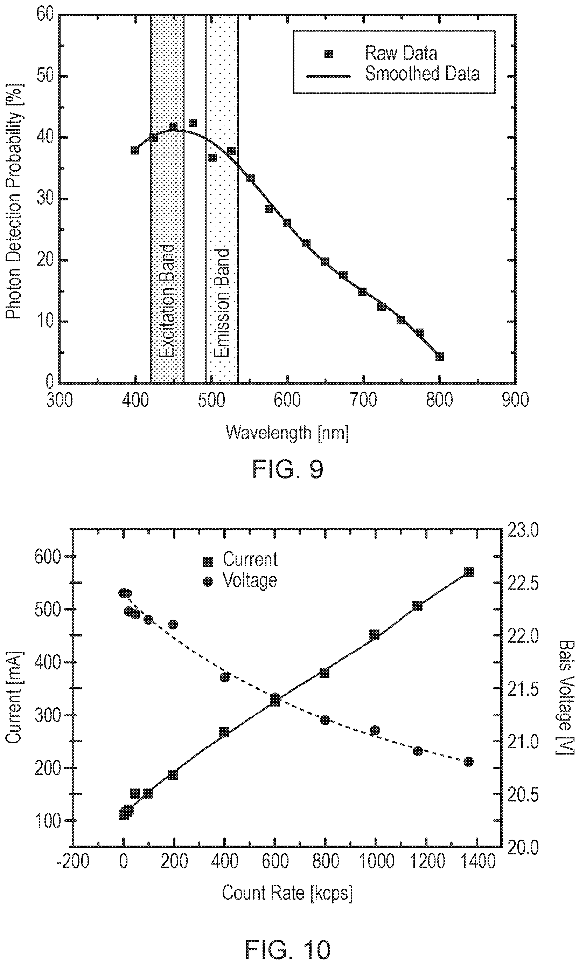

Fig. 9 shows the photon detection probability (PDP) of a single SPAD driven with an excess voltage *Vₑₓ* of 3.05 V measured using a white light source driving an integrating sphere to create a uniform illumination and a monochromator sweeping the light wavelength from 400 to 800 nm. The SPAD was placed at the centre of the monochromator exit port in order to investigate the count rate against wavelength. Due to the non-uniform spectral distribution of the monochromator grating, the results were then remapped against the spectral response measurements obtained from an off-the-shelf BX65 photodiode. As shown in Fig. 9, the SPAD's PDP peaks at 42.4% at 475 nm and is 36.6% at the wavelength of interest (520 nm). The high PDP in the blue-green region makes these SPADs suitable for the autofluorescence detection proposed herein. The pass bands for the illumination signal and the response signal formed by the band pass filters 118, 120 are shown on Fig. 9.

The charge pump's performance was also tested and evaluated experimentally and its performance was optimized to fit the application purpose of the SPAD array. At 1 MΩ load and no capacitive load, the charge pump deliver a maximum high voltage pulse swinging between 33 and 39.7 V using a 1 kHz clock. Adding a capacitive load results in a DC voltage with high voltage ripple. Reducing the ripple voltage is at the expense of the charge pump's current consumption and the delivered current at the output. In order to operate the charge pump at minimum power consumption and to maintain maximum current delivery to the array, the charge pump was set to operate without adding any capacitive load. The delivered voltage and current of the charge pump is function of the clock frequency and the load. A fixed clock frequency 8.2 kHz has been chosen to be the clock of the charge pump as it can be easily derived from a 32 kHz off chip oscillator. In order to simplify the design the charge pump is open loop. Regulation is achieved by a resistor in parallel with the SPAD array load. The only means of regulating the output is by introducing a resistive load to deliver the right bias for the SPAD. At 200 kΩ the charge pump can deliver a high voltage pulses swinging between 17 V and 22.5 V. This pulse has a 50% duty cycle. Therefore, when array is biased by the charge pump, it will only be active when the clock is 22 V.

The response of the integration of the SPAD array and the charge pump was tested by connecting the charge pump output to the bias point of the SPAD array. Fig. 10 shows a measured current and voltage response of the charge pump when connected to the SPAD array as a function of count rate.

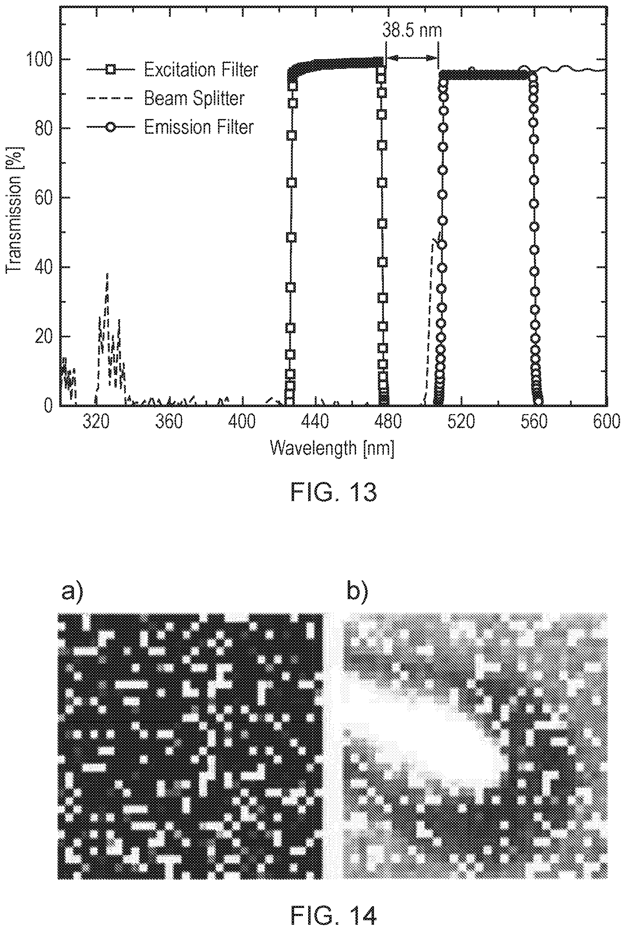

In dark conditions, more than 80% of SPADs are producing an average dark count rate DRC of 11 kcps (see the first image in Fig. 14).

In order to verify that the charge pump is capable of driving the SPAD at high illumination intensity, the array was illuminated with a green LED which has a substantial output at the 520 nm emission wave-length of a commonly used labelling agent (FTIC). At high illumination, each SPADs of one column can reach maximum to 1.4 Mcps. Although, the charge pump drops to 2.08, but still can maintain the SPADs biased above breakdown voltage by *Vₑₓ* 2.08V.

Fig. 11 shows the experimental results of the integration of the charge pump and the array SPAD. The top part of the figure is the high voltage output bias-pulses that are produced by the charge pump. Bottom part shows the generated pulse by a single SPAD of one column of the array at high illumination.

To demonstrate the capability of the integrated chip to perform continue-wave fluorescence imaging, a 10 µM of FITC fluorophore solution was used. By using power addressing generated by an of chip microcontroller, every column was set to be activated for 10 ms. The second image in Fig. 14 shows the drop of the FITC fluorophore solution when place on top of a band pass filter and excited by 470nm blue LED. At 10 ms gating time the imager can perform 3 frames per second (fps). For less sensitive applications, the gating of the imager can be as low as 1 ms and thus can execute up to 30 fps.

Fig. 12 shows an overall timing diagram of the operation of the capsule. As discussed above, the counter gate may be open for a 10ms period to count the SPAD responses. Following the counter gate period, the SPAD array is dormant for a transmission period. This may assist in efficient power sharing within the device.

Fig. 13 shows the spectral characteristic of the interference filter cube in combination with the two band pass filters. It may be seen that the pass bands for the illumination signal and the response signal are separated by a gap of 38.5 nm.

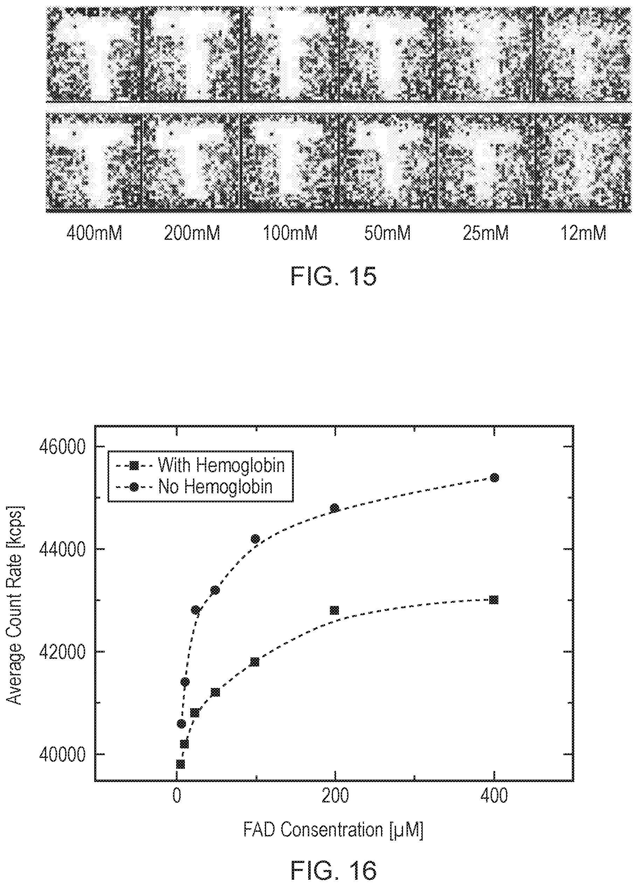

Fig. 15 shows measurement results obtained using cancer phantoms. The top row of images in Fig. 15 show a T-shaped feature captured by the image sensor of the endoscopy capsule. The T-shaped feature was formed by a 2 ml drop of FAD fluorophore solution at different concentrations.

The bottom row of images in Fig. 15 show the same features after adding 0.5 mg of haemoglobin to each one.

Fig. 16 shows the effect on count rate of adding the haemoglobin. These results demonstrate that the present invention may be effective in detected fluorescent labelling agents.

Fig. 17 shows a comparison of a count rate detected from an autofluorescence (i.e. in the absence of labelling agents) emission obtained by illuminating a 4.7 cm² sample of porcine small intestine with a count rate detected from a mirrored reflection response taken in the absence of the interference filter cube.

An integrated high voltage charge pump with a 32×32 SPAD array has been described. A dedicated power management strategy has been adapted to efficiently distribute the limited power delivered by the charge pump. Experimental results show that the charge pump is capable of biasing the SPAD array at 22.5 V at low light conditions. Even at high illumination were SPADs reach to saturation, charge pump can still sustain a reasonably substantial 2.08 V excess voltage over the breakdown voltage, and thus keeping it in Geiger mode. At low light levels relevant for fluorescence detection of cancer the charge pump is expected to effectively maintain a steady biasing for the array SPAD. The integrated chip is incorporated with a means for minimising optical interference in a wireless endoscope fluoresce diagnostic pill.

### REFERENCES

[1] W. K. Hirota, M. J. Zuckerman, D. G. Adler, R. E. Davila, J. Egan, J. A. Leighton, W. A. Qureshi, E. Rajan, R. Fanelli, J. Wheeler-Harbaugh, T. H. Baron, andD.O. Faigel, Standards of Practice Committee, ASGE Guideline: The Role of Endoscopy in the Surveillance of Premalignant Conditions of the Upper GI Tract, Gastrointestinal Endoscopy, vol. 63, no. 4, Apr. 2006, pp. 570-580.
[2] M.Yu, "M2A(TM) capsule endoscopy:Abreakthrough diagnostic tool for small intestine imaging," Gastroenterology Nursing, vol. 25, pp. 24-27, 2002.
[3] M. S. Sachdev and M. K. Ismail, "Capsule endoscopy: A review," South. Med. J., vol. 101, pp. 407-414, 2008.
[4] C. Dongmei, M. Q. H. Meng, W. Haibin, H. Chao, and L. Zhiyong, "A novel strategy to label abnormalities for wireless capsule endoscopy frames sequence," in Proc. IEEE Int. Conf. Inf. Autom., Jun. 2011, pp. 379-383.
[5] L.-M.WongKee Song, S. Banerjee, D. Desilets,D. L.Diehl, F. A. Farraye, V. Kaul, S. R. Kethu, R. S. Kwon, P. Mamula, M. C. Pedrosa, S. A. Rodriguez, and W. M. Tierney, "Autofluorescence imaging," Gastrointestinal Endoscopy, vol. 73, pp. 647-650, 2011.
[6] N. Uedo, K. Higashino, R. Ishihara, Y. Takeuchi, and H. Iishi, "Diagnosis of colonic adenomas by new autofluorescence imaging system: A pilot study," Digestive Endoscopy, vol. 19, pp. S134-S138, 2007.
[7] N. Uedo, R. Ishihara, and H. Iishi, "Autofluorescence Imaging Video-Endoscopy System for Diagnosis of Superficial Gastric Neoplasia," in New Challenges in Gastrointestinal Endoscopy, H. Niwa, H. Tajiri, M. Nakajima, and K. Yasuda, Eds., ed: Springer Japan, 2008, pp. 191-199.
[8] B. Mayinger, M. Jordan, T. Horbach, P. Horner, C. Gerlach, S. Mueller, W. Hohenberger, and E. G. Hahn, "Evaluation of in vivo endoscopic autofluorescence spectroscopy in gastric cancer," Gastrointestinal Endoscopy, vol. 59, pp. 191-198, 2004.
[9] E. Crowell, G. Wang, J. Cox, C. P. Platz, and L. Geng, "Correlation coefficient mapping in fluorescence spectroscopy: Tissue classification for cancer detection," Anal. Chem., vol. 77, pp. 1368-1375, 2005.
[10] B. Mayinger, P. Horner, M. Jordan, C. Gerlach, T. Horbach, W. Hohenberger, and E. G. Hahn, "Endoscopic fluorescence spectroscopy in the upper GI tract for the detection of GI cancer: Initial experience," Amer. J. Gastroenterol., vol. 96, pp. 2616-2621, 2001.
[11] W. Lei, Y. Guang-Zhong, H. Jin, Z. Jinyong, Y. Li, N. Zedong, and D. R. S. Cumming, "A wireless biomedical signal interface system-onchip for body sensor networks," IEEE Trans. Biomed. Circuits Syst., vol. 4, no. 2, pp. 112-117, Apr. 2010.
[12] A. Gallivanoni, I. Rech, and M. Ghioni, "Progress in Quenching Circuits for Single Photon Avalanche Diodes," Nuclear Science, IEEE Transactions on, vol. 57, pp. 3815-3826, 2010.
[13] R. Henderson, G.-F. Dalla-Betta, L. Pancheri, D. Stoppa, and J. Richardson, "Avalanche Photodiodes in Submicron CMOS Technologies for High-Sensitivity Imaging," in Advances in Photodiodes, ed: InTech, 2011.
[14] M. Al-Rawhani, D. Cumming, D. Chitnis, and S. Collins, "Photocurrent dependent response of a SPAD biased by a charge pump," in Circuits and Systems (ISCAS), 2011 IEEE International Symposium on, pp. 789-792.
[15] F. P. T. Samaddar, Charge Pump Circuit Design. NewYork: McGraw-Hill, 2006.
[16] P. Favrat, P. Deval, and M. J. Declercq, "A high-efficiency CMOS voltage doubler," Solid-State Circuits, IEEE Journal of, vol. 33, pp. 410-416, 1998.
[17] Y. TianRui, K. Wing-Hung, and C. Mansun, "Area-efficient CMOS charge pumps for LCD drivers," Solid-State Circuits, IEEE Journal of, vol. 38, pp. 1721-1725, 2003.
[18] S. M. Sze and G. Gibbons, "Effect of junction curvature on breakdown voltage in semiconductors," Solid-State Electron., vol. 9, pp. 831-845, 1966.
[19] Z. Xiao, D. Pantic, and R. S. Popovic, "A new single photon avalanche diode in CMOS high-voltage technology," in Proc. Int. Conf. Solid-State Sensors, Actuators Microsyst., 2007, pp. 1365-1368.
[20] S. Tisa, F. Guerrieri, and F. Zappa, "Variable-load quenching circuit for single-photon avalanche diodes," Opt. Exp., vol. 16, pp. 2232-2244, 2008.
[21] D. Mosconi, D. Stoppa, L. Pancheri, L. Gonzo, and A. Simoni, "CMOS single-photon avalanche diode array for time-resolved fluorescence detection," in Proc. 32nd Eur. Solid-State Circuits Conf., 2006, pp. 564-567.
[22] Matthew B. Sturm, Bishnu P. Joshi, Shaoying Lu, Cyrus Piraka, Supang Khondee, Badih Joseph Elmunzer, Richard S. Kwon, David G. Beer, Henry D. Appelman, Danielle Kim Turgeon, Thomas D. Wang, "Targeted Imaging of Esophageal Neoplasia with a Fluorescently Labeled Peptide: First-in-Human Results", Sci Transl Med 8 May 2013 5:184ra61. [DOI:10.1126/scitranslmed.3004733]

## Claims

1. An endoscopy capsule (100) for detecting fluorescence emitted by biological tissue, wherein :
the endoscopy capsule comprises a housing (102) containing:
a plurality of single photon avalanche diodes (SPADs) arranged in an array (134) to receive incident light from outside the housing,
a light source (110), and
a prismatic optical interference prevention unit (114) comprising an optical beam splitter (116) arranged to define separate optical paths for the output optical signal from the light source and the detected fluorescence signal incident on the array of SPADs.

2. An endoscopy capsule according to claim 1, wherein each SPAD in the array has a breakdown voltage, and wherein the array comprises a plurality of SPAD subsets, each SPAD subset containing one or more SPADs, and
wherein the housing further contains:
a battery (126),
a charge pump (136) connected to receive power from the battery and arranged to generate an output voltage, and
a power management controller arranged to multiplex the output voltage across the plurality of SPAD subsets,
wherein the charge pump is arranged to generate the output voltage at a level that exceeds the breakdown voltage of the one or more SPADs in each SPAD subset.

3. An endoscopy capsule according to claim 2, wherein the array comprises a 32 × 32 matrix of SPADs, and wherein each SPAD subset is a column or row of 32 SPADs.

4. An endoscopy capsule according to claim 2 or 3, wherein the power management controller comprises a multiplexer (142) and a plurality of conditioning circuits (152), each conditioning circuit being arranged to supply the output voltage as a bias voltage to the one or more SPADs in a respective SPAD subset.

5. An endoscopy capsule according to claim 4, wherein each conditioning circuit includes a time gated voltage divider for supplying the bias voltage to the one or more SPADs in its respective SPAD subset.

6. An endoscopy capsule according to any preceding claim, wherein each SPAD is mounted on a pixel unit that including a photon strike pulse detector arranged to output a count signal for each detected photon strike pulse, wherein the photon strike pulse detector includes an inverting comparator with a controllable trigger threshold.

7. An endoscopy capsule according to any preceding claim including:
a detector for outputting an detection signal indicative of a number of photon detection events in the array of SPADs, and
a transmitter (128) for wirelessly communicating the detection signal out of the capsule.

8. An endoscopy capsule according to claim 7, wherein the detector comprises a plurality of counters (140), each counter being arranged to count the photon strike pulses from a single SPAD in each respective SPAD subset.

9. An endoscopy capsule according to claim 8, wherein the detector include a counter multiplexer for gathering the content of the plurality of counters into a single detection signal.

10. An endoscopy capsule according to any preceding claim, wherein the optical interference prevention unit comprises a first optical band pass filter on the output optical signal path from the light source and a second optical band pass filter on an incident optical signal path for the array of SPADS, wherein the pass band of the first optical band pass filter is separated from the pass band of the second optical band pass filter.

11. An endoscopy capsule according to any preceding claim, wherein the optical interference prevention unit comprises a common input/output aperture for the output optical signal path from the light source and the incident optical signal path for the array of SPADS.

12. An endoscopy capsule according to claim 10, including a lens (106) arranged to direct incident light through the common input/output aperture.

13. An endoscopy capsule according to claim 12, wherein the lens forms part of the housing.

14. An endoscopy capsule according to any preceding claim, wherein the light source is a light emitting diode.

15. An endoscopy capsule according to any preceding claim, wherein the housing is transparent.

## Patentansprüche

1. Endoskopiekapsel (100) zum Detektieren von Fluoreszenz, die von biologischem Gewebe emittiert wird,
wobei die Endoskopiekapsel ein Gehäuse (102) umfasst, das Folgendes enthält:
eine Vielzahl von Einzel-Photon-Lawinendioden (SPADs), die in einer Matrix (134) angeordnet sind, um von außerhalb des Gehäuses einfallendes Licht aufzunehmen,
eine Lichtquelle (110), und
eine prismatische optische Interferenzverhinderungseinheit (114), die einen optischen Strahlteiler (116) aufweist, der so angeordnet ist, dass er separate optische Pfade für das optische Ausgangssignal von der Lichtquelle und das auf die Matrix von SPADs einfallende, detektierte Fluoreszenzsignal definiert.

2. Endoskopiekapsel gemäß Anspruch 1, wobei jede SPAD in der Matrix eine Durchbruchsspannung aufweist, und wobei die Matrix eine Vielzahl von SPAD-Untergruppen umfasst, worin jede SPAD-Untergruppe eine oder mehrere SPADs enthält, und
wobei das Gehäuse ferner Folgendes enthält:
eine Batterie (126),
eine Ladungspumpe (136), die angeschlossen ist, um Leistung aus der Batterie aufzunehmen und so angeordnet ist, dass sie eine Ausgangsspannung erzeugt, und
eine Leistungs-Management-Steuerungseinheit, die so angeordnet ist, dass sie die Ausgangsspannung an der Vielzahl von SPAD-Untergruppen multiplext,
wobei die Ladungspumpe ausgestaltet ist, die Ausgangsspannung in einem Ausmaß zu erzeugen, das die Durchbruchsspannung der einen oder der mehreren SPADs in jeder SPAD-Untergruppe überschreitet.

3. Endoskopiekapsel gemäß Anspruch 2, wobei die Matrix eine 32 x 32 -Matrix von SPADs umfasst, und wobei jede SPAD-Untergruppe eine Spalte oder Zeile von 32 SPADs ist.

4. Endoskopiekapsel gemäß Anspruch 2 oder 3,
wobei die Leistungs-Management-Steuerungseinheit einen Multiplexer (142) und eine Vielzahl von Konditionierschaltungen (152) umfasst, wobei jede Konditionierschaltung so angeordnet ist, dass sie die Ausgangsspannung der einen oder den mehreren SPADs in einer jeweiligen SPAD-Untergruppe als eine Vorspannung zuführt.

5. Endoskopiekapsel gemäß Anspruch 4, wobei jede Konditionierschaltung einen Zeit-gattergesteuerten Spannungsteiler zum Zuführen der Vorspannung zu der einen oder den mehreren SPADs in deren jeweiligen SPAD-Untergruppe umfasst.

6. Endoskopiekapsel gemäß einem der vorangegangenen Ansprüche, wobei jede SPAD auf einer Pixeleinheit befestigt ist, die einen Photonenaufprallimpulsdetektor umfasst, der so angeordnet ist, dass er ein Zählsignal für jeden detektierten Photonenaufprallimpuls ausgibt, wobei der Photonenaufprallimpulsdetektor einen invertierenden Komparator mit einer steuerbaren Auslöseschwelle umfasst.

7. Endoskopiekapsel gemäß einem vorangegangenen Anspruch, aufweisend:
einen Detektor zum Ausgeben eines Detektionssignals, das eine Anzahl von Photonendetektionsereignissen in der Matrix von SPADs anzeigt, und
einen Sender (128), um das Detektionssignal drahtlos aus der Kapsel heraus zu übertragen.

8. Endoskopiekapsel gemäß Anspruch 7, wobei der Detektor eine Vielzahl von Zählern (140) umfasst, wobei jeder Zähler ausgelegt ist, die Photonenaufprallimpulse von einer Einzel-SPAD in jeder jeweiligen SPAD-Untergruppe zu zählen.

9. Endoskopiekapsel gemäß Anspruch 8, wobei der Detektor einen Zähler-Multiplexer umfasst, um den Inhalt der Vielzahl von Zählern in einem Einzel-Detektionssignal zusammenzufassen.

10. Endoskopiekapsel gemäß einem der vorangegangenen Ansprüche, wobei die optische Interferenzverhinderungseinheit einen ersten optischen Bandpassfilter auf dem optischen Ausgangssignalpfad von der Lichtquelle und einen zweiten optischen Bandpassfilter auf einem optischen Eingangssignalpfad für die Matrix von SPADs umfasst, wobei das Passband des ersten optischen Bandpassfilters vom Passband des zweiten optischen Bandpassfilters getrennt ist.

11. Endoskopiekapsel gemäß einem der vorangegangenen Ansprüche, wobei die optische Interferenzverhinderungseinheit eine gemeinsame Eingangs-/AusgangsÖffnung für den optischen Ausgangssignalpfad von der Lichtquelle und den optischen Eingangssignalpfad für die Matrix von SPADs umfasst.

12. Endoskopiekapsel gemäß Anspruch 10, umfassend eine Linse (106), die so angeordnet ist, dass sie einfallendes Licht durch die gemeinsame Eingangs-/Ausgangs-Öffnung hindurch leitet.

13. Endoskopiekapsel gemäß Anspruch 12, wobei die Linse einen Teil des Gehäuses bildet.

14. Endoskopiekapsel gemäß einem der vorangegangenen Ansprüche, wobei die Lichtquelle eine lichtemittierende Diode ist.

15. Endoskopiekapsel gemäß einem der vorangegangenen Ansprüche, wobei das Gehäuse transparent ist.

## Revendications

1. Capsule d'endoscopie (100) pour détecter une fluorescence émise par un tissu biologique, dans laquelle :
la capsule d'endoscopie comprend un logement (102) contenant :
une pluralité de diodes avalanche à photon unique (SPAD) disposées en un réseau (134) pour recevoir de la lumière incidente provenant de l'extérieur du logement,
une source de lumière (110), et
une unité de prévention d'interférence optique prismatique (114) comprenant un séparateur de faisceau optique (116) conçu pour définir des trajets optiques séparés pour le signal optique de sortie provenant de la source de lumière et le signal de fluorescence détecté frappant le réseau de SPAD.

2. Capsule d'endoscopie selon la revendication 1, dans laquelle chaque SPAD dans le réseau a une tension de claquage, et dans laquelle le réseau comprend une pluralité de sous-ensembles de SPAD, chaque sous-ensemble de SPAD contenant une ou plusieurs SPAD, et
dans laquelle le logement contient en outre :
une batterie (126),
une pompe de charge (136) connectée pour recevoir de l'énergie de la batterie et conçue pour générer une tension de sortie, et
un dispositif de commande de gestion d'énergie conçu pour multiplexer la tension de sortie à travers la pluralité de sous-ensembles de SPAD,
dans laquelle la pompe de charge est conçue pour générer la tension de sortie à un niveau qui dépasse la tension de claquage des une ou plusieurs SPAD dans chaque sous-ensemble de SPAD.

3. Capsule d'endoscopie selon la revendication 2, dans laquelle le réseau comprend une matrice 32 x 32 de SPAD, et dans laquelle chaque sous-ensemble de SPAD est une colonne ou une rangée de 32 SPAD.

4. Capsule d'endoscopie selon la revendication 2 ou 3, dans laquelle le dispositif de commande de gestion d'énergie comprend un multiplexeur (142) et une pluralité de circuits de conditionnement (152), chaque circuit de conditionnement étant agencé pour alimenter la tension de sortie sous forme d'une tension de polarisation aux une ou plusieurs SPAD dans un sous-ensemble de SPAD respectif.

5. Capsule d'endoscopie selon la revendication 4, dans laquelle chaque circuit de conditionnement comprend un diviseur de tension à déclenchement périodique pour alimenter la tension de polarisation aux une ou plusieurs SPAD dans son sous-ensemble de SPAD respectif.

6. Capsule d'endoscopie selon l'une quelconque des revendications précédentes, dans laquelle chaque SPAD est montée sur une unité de pixel comprenant un détecteur d'impulsions de bombardement de photons conçu pour délivrer en sortie un signal de comptage pour chaque impulsion de bombardement de photons détectée, le détecteur d'impulsions de bombardement de photons comprenant un comparateur inverseur avec un seuil de déclenchement contrôlable.

7. Capsule d'endoscopie selon l'une quelconque des revendications précédentes comprenant :
un détecteur pour délivrer en sortie un signal de détection indiquant un nombre d'événements de détection de photons dans le réseau de SPAD, et
un émetteur (128) pour communiquer sans fil le signal de détection hors de la capsule.

8. Capsule d'endoscopie selon la revendication 7, dans laquelle le détecteur comprend une pluralité de compteurs (140), chaque compteur étant agencé pour compter les impulsions de bombardement de photons provenant d'une SPAD unique dans chaque sous-ensemble de SPAD respectif.

9. Capsule d'endoscopie selon la revendication 8, dans laquelle le détecteur comprend un compteur multiplexeur pour regrouper le contenu de la pluralité de compteurs en un signal de détection unique.

10. Capsule d'endoscopie selon l'une quelconque des revendications précédentes, dans laquelle l'unité de prévention d'interférence optique comprend un premier filtre passe-bande optique sur le trajet de signal optique de sortie depuis la source de lumière et un second filtre passe-bande optique sur un trajet de signal optique incident pour le réseau de SPAD, dans lequel la bande passante du premier filtre passe-bande optique est séparée de la bande passante du second filtre passe-bande optique.

11. Capsule d'endoscopie selon l'une quelconque des revendications précédentes, dans laquelle l'unité de prévention d'interférence optique comprend une ouverture d'entrée/sortie commune pour le trajet de signal optique de sortie depuis la source lumineuse et le trajet de signal optique incident pour le réseau de SPAD.

12. Capsule d'endoscopie selon la revendication 10, comprenant une lentille (106) agencée pour diriger une lumière incidente à travers l'ouverture d'entrée/sortie commune.

13. Capsule d'endoscopie selon la revendication 12, dans laquelle la lentille fait partie du logement.

14. Capsule d'endoscopie selon l'une quelconque des revendications précédentes, dans laquelle la source de lumière est une diode électroluminescente.

15. Capsule d'endoscopie selon l'une quelconque des revendications précédentes, dans laquelle le logement est transparent.
